# EUROPEAN PATENT SPECIFICATION

(11) **EP 0 731 352 B1**
(45) Date of publication and mention of the grant of the patent: **12.05.1999**
(21) Application number: 96301415.4
(22) Date of filing: 01.03.1996
(51) Int. Cl.: G01N 27/60

(54) **Plastics identification**
Kunststoffidentifizierung
Identification de plastiques

(30) Priority: 07.03.1995 GB 9504560
(43) Date of publication of application: 11.09.1996
(73) Proprietor: FORD MOTOR COMPANY LIMITED, Brentwood Essex (GB); FORD FRANCE S. A., 92506 Rueil-Malmaison Cédex (FR); FORD-WERKE AKTIENGESELLSCHAFT, 50735 Köln (DE)
(72) Inventor: Mucci,Peter Edmund Reuben, Durley Southampton SO3 2AD (GB); Amner, John Arthur, Rochford Essex SS4 1SR (GB); Gentle, Derek Francis, Danbury Essex CM3 4EH (GB); Whyard, Dennis Edward, Fareham Hants PO14 4JZ (GB)
(74) Representative: Messulam, Alec Moses

(56) References cited:
- WO-A-91/19204
- WO-A-94/17402

## Description

This invention relates to a device for identifying different plastics materials. The device is particularly (but not exclusively) suitable for use in separating plastics components into chemically similar groups, as a first stage in, for example, the recycling of the plastics materials. In this specification, "plastics" includes both thermoplastic and thermoset materials, whether in the form of solid or foamed material and also includes natural and synthetic rubbers and any composite material which is a composition of any of these materials. The invention is also applicable to such materials in which additives (plastics or non plastics) have been included.

International Patent Application WO 91/19204 discloses a device and method for measuring low amplitude signals on a moving strip material. The strip material runs over a rotatable roller which has a measuring element for picking up low-amplitude signals. The device includes a conductive brush for eliminating residual charge on the measuring element as the element makes contact with the brush during each rotation.

Our International Patent Application WO 94/17402 describes and claims both a method and an apparatus for identifying different plastics material, and the content of that specification is incorporated herein by reference. This application relates to a device and method for using triboelectricity to identify particular plastics materials which produce a characteristic charge polarity when rubbed against another known material. Different reference materials are rotationally mounted so that they can be rubbed against the material to be identified to create electrostatic charges which are then processed by a logic circuit to identify the nature of the sample.

The present invention provides an improved device for use in the method of our earlier invention.

According to the present invention, there is provided a device for identifying plastics materials, the device comprising an electrically isolated and electrostatically chargeable detector head mounted on an insulating support, and a head discharging mechanism to discharge the head prior to recharging the head by rubbing it against a plastics material sample, the head discharging mechanism comprising a flexible, earthed conductor mounted relative to the head; characterised in that the conductor is mounted on a translationally displaceable mounting such that the conductor can be moved past the head whereupon the conductor makes electrically conductive contact with the head to discharge the head, the conductor being thereafter movable into contact with the insulating support to enable the head to be charged by rubbing it against a sample.

The device preferably has more than one detector head, and in a preferred embodiment the detector head has a surface of a plastics material which can be rubbed against the sample, with the plastics material surface being itself in contact with a support of an electrically conductive material which does not come into direct contact with the sample surface, the conductive support being connected to means for measuring the polarity of charge induced on the support.

The flexible, earthed conductor may be a conductive brush or brushes which wipe over the heads, but is preferably a diaphragm made of a conductive, flexible material.

In operation, displacement of the diaphragm first makes electrical contact with the plastics material surface, and then with the conductive support, before taking up position against the insulating support.

The head or heads preferably project from one end of a housing of the device, and the diaphragm is supported on a collar which is displaceable in an axial direction relative to the direction in which the heads project from the housing. The collar is preferably displaceable against a force which normally urges the diaphragm into a forward position in which it discharges the head or heads. The diaphragm may either be directly earthed, or may be connected to the collar which itself is conductive and is earthed, to allow any charge on the head to flow away through the diaphragm to earth.

The diaphragm may include a forwardly projecting portion arranged such that when the device is pressed against a plastics material sample, the projection first makes contact with the sample, and as the device is moved against the sample, the projection slides back into the housing and causes the diaphragm to displace relative to the head or heads to first discharge the head and then to come to rest on the insulating support, in which position the device can be used to conduct a new test on an unidentified plastics material sample.

The diaphragm can be made from a conducting rubber material which is suitably slit where it will come into contact with the head or heads, so that as the diaphragm is displaced, the head or heads will push through the slit area of the diaphragm and the parts of the diaphragm where the slits are positioned will rub against the sides of the head or heads to make electrical contact through which any charge on the head or heads can be discharged.

The device described here is related to the device previously described in our co-pending European patent application 95115629.8, now published as EP 0 767 374. This application relates to a device for identifying plastics materials, the device having detector heads which have a surface of a plastics material which can be rubbed against a sample. The plastics material surface is itself in contact with a support of an electrically conductive material which does not come into direct contact with the sample surface. When the plastics surface of the detector head is rubbed against a sample, a charge is created on the conductive support which is measured to produce an output indicating the nature of the sample.

The invention will now be further described, by way of example, with reference to the accompanying drawings, in which:
Figure 1 is a perspective view of a device in accordance with the invention;
Figures 2, 3, 4 and 5 show sequential stages in the operation of the device;
   and
Figure 6 is a scrap view showing a head projecting through the diaphragm.

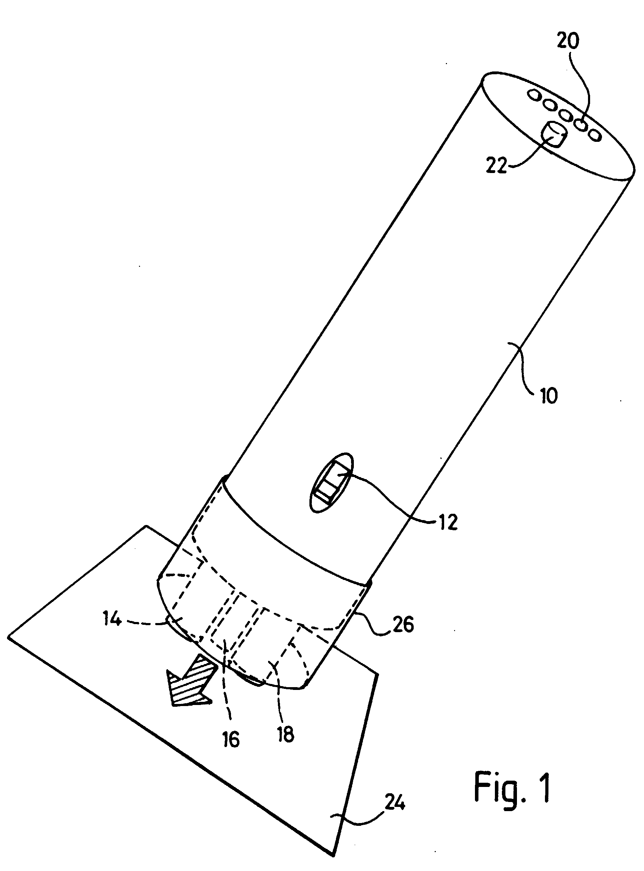

The device shown in Figure 1 has a housing 10 of cylindrical form of a size which can be easily held in the hand. Although a simple cylindrical form is shown in Figure 1, the housing could be shaped to make it comfortable to hold in the hand, in a known way.

The device has an ON/OFF switch 12 and three detector heads 14, 16, 18 at one end. At the other end, the housing has five indicator lamps 20 and a reset button 22. In the device shown, with three detector heads, it is possible to separately identify four different plastics types, so four of the lights relate to specific plastics types and in use an appropriate one of the lights will light up in accordance with the plastic type detected. The fifth light indicates a failed reading and tells the operator that the device must be reapplied to the sample surface and another reading taken.

Figure 1 shows a sample surface against which the device is to be rubbed at 24.

When the heads are brought into frictional contact with the surface of the sample 24, by rubbing one against the other, electrostatic charge is generated on the face of each head by triboelectrification. The magnitude and polarity of the electrostatic charge generated in this way will vary depending on the material of the sample surface. The charge generated in this way can be measured and used to identify the nature of the plastics material sample 24, as described in our previous applications WO94/17402 and EP 0 767 374.

Before a new sample can be tested using this device, it is necessary to discharge any charge remaining on the heads 14, 16, 18 and resulting from previous operation of the device, or otherwise.

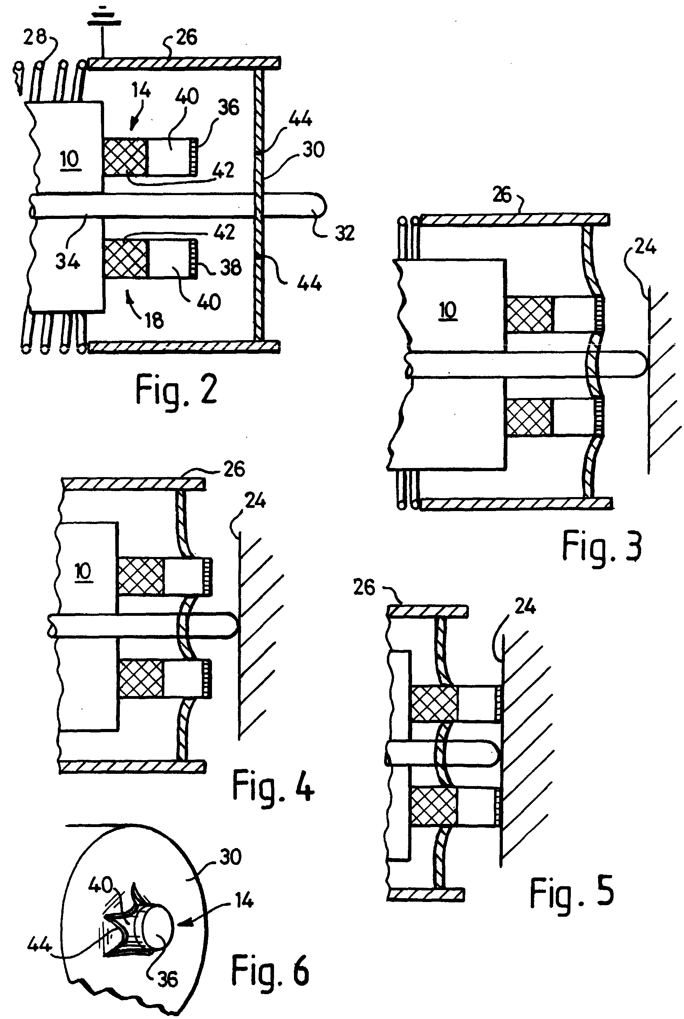

To ensure that the heads are properly discharged, each time a reading is to be taken, the arrangement shown in Figures 2-6 is used.

A collar 26 is fitted over the bottom end of the cylindrical housing 10 and is axially movable along the housing against the force of a restoring spring 28 which will restore the collar to the position shown in Figure 2 in the absence of any external applied force. The collar 26 supports a flexible conductive diaphragm 30 which extends across the end of the device and in front of the heads 14, 18. The diaphragm is fixed to a projecting pin 32 which is guided for sliding movement at 34 inside the housing of the device 10.

Each head 14, 18 has a plastics disk 36, 38 mounted, for example by adhesive, on the end of a conductive cylinder 40. The conductive cylinders 40 are mounted on an insulting support boss 42. The materials of the plastics disks 36 and 38 will be different from one another and will be chosen in accordance with the particular plastics to be identified.

In operation, the device is brought into contact with the sample surface 24, and the first part to make contact is the tip of the projecting pin 32. The device is pressed against the sample so that the pin 32 retracts into the housing 10 and the spring 28 is compressed. As this happens initially, the diaphragm, which is split at 44 where it overlies the pins 14, 18 makes electrically contact with the ends of the pins so that any charge remaining on the plastics disks 36, 38 is discharged to earth.

As the device is pressed further against the sample, the diaphragm passes along the length of the pins 14, 18 and next makes electrical conducting contact with the conductive cylinders 40, so that these cylinders are also discharged. Figure 6 shows the way in which one of the heads 14 projects through the diaphragm 30 at this stage. The way in which the diaphragm is slit at 44 will be apparent from this figure.

Further movement of the device towards the surface 24 results in complete retraction of the pin 32 when the plastics disks 36, 38 come into contact with the surface 24. As will be seen from Figure 5, in this condition, the diaphragm 30 is in electrical contact only with the insulating bosses 42 so that any charge built up on the heads 14, 18 with the diaphragm in this position will not be dissipated. The charge can be built up and measured to enable a reading to be taken.

However, as soon as the device is moved away from the surface 24, the restoring force of the spring 28 will cause the diaphragm to move forward to pass the conductive portions 40, 36 and 38 to perform a discharging action. This discharging action will be repeated next time the device is used against another sample surface 24.

Although a diaphragm 30 has been shown in the accompanying drawings, other conductive components could be used in place of this diaphragm. For example a series of brushes could wipe over the heads 14, 18. These brushes could be mounted on the projecting pin 32, and the pin itself could be spring loaded so that a sleeve 26 can be dispensed with. The skilled man will understand that other arrangements which produce the same effect of discharging the heads automatically on bringing them into contact with the sample surface could alternatively be used.

Furthermore, the ends of the heads 14 and 18 could be curved and/or domed to assist movement of the diaphragm over the heads and to improve electrical, conducting contact between the diaphragm and the heads.

## Claims

1. A device for identifying plastics materials, the device comprising an electrically isolated and electrostatically chargeable detector head (14, 16, 18) mounted on an insulating support (42), and a head discharging mechanism to discharge the head prior to recharging the head by rubbing it against a plastics material sample, the head discharging mechanism comprising a flexible, earthed conductor (30) mounted relative to the head (14, 16, 18);
characterised in that the conductor (30) is mounted on a translationally displaceable mounting (26) such that the conductor (30) can be moved past the head (14, 16, 18) whereupon the conductor (30) makes electrically conductive contact with the head (14, 16, 18) to discharge the head (14, 16, 18), the conductor (30) being thereafter movable into contact with the insulating support (42) to enable the head (14, 16, 18) to be charged by rubbing it against a sample.

2. A device as claimed in Claim 1, which has more than one detector head (14, 16, 18).

3. A device as claimed in Claim 1 or Claim 2, wherein the detector head (14, 16, 18) has a surface of a plastics material (36, 38) which can be rubbed against the sample, with the plastics material surface being itself in contact with a support (40) of an electrically conductive material which does not come into direct contact with the sample surface, the conductive support (40) being connected to means for measuring the polarity of charge induced on the support.

4. A device as claimed in any preceding claim, wherein the flexible, earthed conductor is a diaphragm (30) made of a conductive, flexible material.

5. A device as claimed in Claim 4, wherein the head or heads (14, 16, 18) project from one end of a housing (10) of the device, and the diaphragm (30) is supported on a collar (26) which is displaceable in an axial direction relative to the direction in which the heads (14, 16, 18) project from the housing (10).

6. A device as claimed in Claim 5, wherein the collar (26) is displaceable against a force which normally urges the diaphragm (30) into a forward position in which it discharges the head or heads (14, 16, 18).

7. A device as claimed in Claim 5 or Claim 6, wherein the diaphragm (30) is connected to the collar (26) which itself is conductive and is earthed, to allow any charge on the head to flow away through the diaphragm (30) to earth.

8. A device as claimed in any one of Claims 4 to 7, wherein the diaphragm (30) includes a forwardly projecting portion (32) arranged such that when the device is pressed against a plastics material sample, the projection (32) first makes contact with the sample, and as the device is moved against the sample, the projection (32) slides back into the housing (10) and causes the diaphragm (30) to displace relative to the head or heads (14, 16, 18) to first discharge the head and then to come to rest on the insulating support (42), in which position the device can be used to conduct a new test on an unidentified plastics material sample.

9. A device as claimed in any one of Claims 4 to 8, wherein the diaphragm (30) is made from a conducting rubber material which is slit where it will come into contact with the head or heads (14, 16, 18), so that as the diaphragm (30) is displaced, the head or heads (14, 16, 18) will push through the slit area (44) of the diaphragm (30) and the parts of the diaphragm (30) where the slits are positioned will rub against the sides of the head or heads (14, 16, 18) to make electrical contact through which any charge on the head or heads can be discharged.

## Patentansprüche

1. Eine Vorrichtung zur Identifizierung von Kunststoffmaterialien, wobei die Vorrichtung einen elektrisch isolierten und elektrostatisch aufladbaren Detektorkopf (14, 16, 18) umfaßt, der auf einem isolierenden Träger (42) angebracht ist; und einen Mechanismus, um den Kopf zu entladen, bevor der Kopf durch Reiben an einer Probe aus Kunststoffmaterial wieder aufgeladen wird; wobei der Mechanismus zur Entladung des Kopfes einen flexiblen, geerdeten Leiter (30) umfaßt, der relativ zum Kopf (14, 16, 18) angebracht ist;
dadurch gekennzeichnet, daß der Leiter (30) auf einer parallel verschiebbaren Halterung (26) angebracht ist, so daß der Leiter (30) am Kopf (14, 16, 18) vorbei bewegt werden kann, woraufhin der Leiter (30) einen elektrisch leitenden Kontakt mit dem Kopf (14, 16, 18) herstellt, um den Kopf (14, 16, 18) zu entladen; und der Leiter (30) danach in Kontakt mit dem isolierenden Träger (42) gebracht werden kann, was die Aufladung des Kopfes (14, 16, 18) ermöglicht, indem er an einer Probe gerieben wird.

2. Eine Vorrichtung nach Anspruch 1, die über mehr als einen Detektorkopf (14, 16, 18) verfügt.

3. Eine Vorrichtung nach Anspruch 1 oder Anspruch 2, worin der Detektorkopf (14, 16, 18) eine Oberfläche aus einem Kunststoffmaterial (36, 38) besitzt, die an der Probe gerieben werden kann, wobei die Oberfläche des Kunststoffmaterials selbst in Kontakt mit einem Träger (40) aus einem elektrisch leitfähigen Material steht, welcher nicht in direkten Kontakt mit der Probenoberfläche kommt; und wobei der elektrisch leitende Träger (40) an eine Vorrichtung angeschlossen ist, um die Polarität der auf dem Träger induzierten Ladung zu messen.

4. Eine Vorrichtung nach einem der vorangegangenen Ansprüche, worin der flexible, geerdete Leiter eine aus einem leitfähigen, flexiblen Material gefertigte Membran (30) ist.

5. Eine Vorrichtung nach Anspruch 4, worin der Kopf oder die Köpfe (14, 16, 18) aus einem Ende des Gehäuses (10) der Vorrichtung herausragen, und worin die Membran (30) von einer Manschette (26) getragen wird, welche in einer Richtung parallel zu der Richtung verschoben werden kann, in der die Köpfe (14, 16, 18) aus dem Gehäuse (10) ragen.

6. Eine Vorrichtung nach Anspruch 5, worin die Manschette (26) gegen eine Kraft verschoben werden kann, welche die Membran (30) normalerweise in eine vordere Position zwingt, in der sie den Kopf oder die Köpfe (14, 16, 18) entlädt.

7. Eine Vorrichtung nach Anspruch 5 oder Anspruch 6, worin die Membran (30) an die Manschette (26) angeschlossen ist, welche selbst leitfähig und geerdet ist, so daß jede Ladung auf dem Kopf durch die Membran (30) an die Masse abfließen kann.

8. Eine Vorrichtung nacheinem der Ansprüche 4 bis 7, worin die Membran (30) einen nach vorne vorspringenden Teil (32) umfaßt, der so angeordnet ist, daß der Vorsprung (32) zuerst mit der Probe in Kontakt tritt, wenn die Vorrichtung gegen eine Probe aus Kunststoffmaterial gedrückt wird; wobei dieser Vorsprung (32), während die Vorrichtung gegen die Probe bewegt wird, in das Gehäuse (10) zurückgleitet und bewirkt, daß sich die Membran (30) bezüglich des Kopfes oder der Köpfe (14, 16, 18) bewegt, um den Kopf erst zu entladen und dann an dem isolierenden Träger (42) zum Stillstand zu kommen; in welcher Stellung die Vorrichtung zur Durchführung einer neuen Prüfung an einer Probe eines nicht identifizierten Kunststoffmaterials verwendet werden kann.

9. Eine Vorrichtung nach einem der Ansprüche 4 bis 8, worin die Membran (30) aus einem leitenden Gummimaterial hergestellt ist, das dort, wo es mit dem Kopf oder den Köpfen (14, 16, 18) in Kontakt kommt geschlitzt ist, so daß der Kopf oder die Köpfe (14, 16, 18) durch den geschlitzen Bereich der Membran (30) drücken und die Stellen der Membran (30), an denen die Schlitze angebracht sind, an den Seiten des Kopfes oder der Köpfe (14, 16, 18) reiben, um einen elektrischen Kontakt herzustellen, durch den jede Ladung auf dem Kopf oder den Köpfen entladen werden kann, wenn die Membran (30) verschoben wird.

## Revendications

1. Dispositif d'identification de matériaux de matières plastiques, le dispositif comprenant une tête de détecteur électriquement isolée et pouvant être chargée de façon électrostatique (14, 16, 18) montée sur un support isolant (42), et un mécanisme de décharge de tête afin de décharger la tête avant de recharger la tête en la frottant sur un échantillon d'un matériau de matière plastique, le mécanisme de décharge de la tête comprenant un élément conducteur (30) souple mis à la masse monté par rapport à la tête (14, 16, 18),
caractérisé en ce que l'élément conducteur (30) est monté sur un support pouvant être déplacé selon un mouvement de translation (26) de sorte que l'élément conducteur (30) puisse être déplacé le long de la tête (14, 16, 18) à la suite de quoi l'élément conducteur (30) réalise un contact électriquement conducteur avec la tête (14, 16, 18) afin de décharger la tête (14, 16, 18), l'élément conducteur (30) étant ensuite déplacé jusqu'en contact avec le support isolant (42) afin de permettre à la tête (14, 16, 18) d'être chargée en la frottant sur un échantillon.

2. Dispositif selon la revendication 1, qui comporte plus d'une tête de détecteur (14, 16, 18).

3. Dispositif selon la revendication 1 ou la revendication 2, dans lequel la tête de détecteur (14, 16, 18) comporte une surface d'un matériau de matière plastique (36, 38) qui peut être frottée sur l'échantillon, la surface du matériau de matière plastique étant elle-même en contact avec un support (40) d'un matériau électriquement conducteur qui ne vient pas en contact direct avec la surface de l'échantillon, le support conducteur (40) étant relié à un moyen de mesure de la polarité de la charge induite sur le support.

4. Dispositif selon l'une quelconque des revendications précédentes, dans lequel l'élément conducteur souple mis à la masse est un diaphragme (30) fait d'un matériau souple conducteur.

5. Dispositif selon la revendication 4, dans lequel la tête ou les têtes (14, 16, 18) font saillie à partir d'une première extrémité d'un corps (10) du dispositif, et le diaphragme (30) est supporté sur un manchon (26) qui peut être déplacé selon une direction axiale par rapport à la direction dans laquelle les têtes (14, 16, 18) font saillie à partir du corps (10).

6. Dispositif selon la revendication 5, dans lequel le manchon (26) peut être déplacé en s'opposant à une force qui pousse normalement le diaphragme (30) dans une position vers l'avant dans laquelle il décharge la tête ou les têtes (14, 16, 18).

7. Dispositif selon la revendication 5 ou la revendication 6, dans lequel le diaphragme (30) est relié au manchon (26) qui lui-même est conducteur et est mis à la masse, afin de permettre à toute charge se trouvant sur la tête de circuler par l'intermédiaire du diaphragme (30) vers la masse.

8. Dispositif selon l'une quelconque des revendications 4 à 7, dans lequel le diaphragme (30) comprend une partie faisant saillie vers l'avant (32) agencée de façon que lorsque le dispositif est pressé contre un échantillon d'un matériau de matière plastique, la saillie (32) réalise tout d'abord un contact avec l'échantillon, et lorsque le dispositif est déplacé contre l'échantillon, la saillie (32) se rétracte à l'intérieur du corps (10) et amène le diaphragme (30) à se déplacer par rapport à la tête ou aux têtes (14, 16, 18) afin de décharger tout d'abord la tête et ensuite venir au repos sur le support isolant (42), position dans laquelle le dispositif peut être utilisé pour réaliser un nouveau test sur un échantillon d'un matériau de matière plastique non identifié.

9. Dispositif selon l'une quelconque des revendications 4 à 8, dans lequel le diaphragme (30) est fait d'un matériau de caoutchouc conducteur qui est fendu à l'endroit où il viendra en contact avec la tête ou les têtes (14, 16, 18), de sorte que lorsque le diaphragme (30) sera déplacé, la tête ou les têtes (14, 16, 18) forceront un passage au travers de la zone fendue (44) du diaphragme (30), et les parties du diaphragme (30) où les fentes sont positionnées frotteront le long des côtés de la tête ou des têtes (14, 16, 18) afin de réaliser un contact électrique grâce auquel toute charge quelconque sur la tête ou les têtes peut être déchargée.
